# EUROPÄISCHE PATENTSCHRIFT

(11) **EP 3 988 064 B1**
(45) Veröffentlichungstag und Bekanntmachung des Hinweises auf die Patenterteilung: **22.03.2023**
(21) Anmeldenummer: 20203906.1
(22) Anmeldetag: 26.10.2020
(51) Int. Cl.: A61F 13/12, A61L 15/08

(54) **VERFAHREN ZUR VORBEREITUNG VON GIPSBINDEN, SYSTEM ZUR DURCHFÜHRUNG DES VERFAHRENS**
METHOD FOR PREPARING PLASTER BANDAGES, SYSTEM FOR CARRYING OUT THE METHOD
PROCÉDÉ DE PRÉPARATION DE BANDES DE PLÂTRE, SYSTÈME DE MISE EN OEUVRE DU PROCÉDÉ

(43) Veröffentlichungstag der Anmeldung: 27.04.2022
(73) Patentinhaber: Klose, Thomas, 56068 Koblenz (DE); Schmidt, Sandra, 56076 Koblenz (DE)
(72) Erfinder: Klose, Thomas, Dr., 56068 Koblenz (DE)
(74) Vertreter: Bernsmann, Falk

(56) Entgegenhaltungen:
- DE-U1- 20 105 609
- FR-A- 1 193 115
- US-A1- 2015 073 930
- US-A1- 2020 329 849
- US-B1- 7 117 543

## Beschreibung

### Technisches Gebiet

Die Erfindung betrifft ein Verfahren zur Vorbereitung von Gipsbinden für einen Gipsverband aus einer Mehrzahl von Gipsbinden-Lagen für eine menschliche Nase und ein System zur Durchführung des Verfahrens.

### Stand der Technik

Aus dem Stand der Technik ist es bekannt, eine menschliche Nase zur Ruhigstellung mit einem Gipsverband zu versehen, beispielsweise nach einem Nasenbeinbruch oder einer Nasenoperation. Nachteilig an einem üblichen, aus Gipsbinden hergestellten Gipsverband ist, dass die Gipsbinden zunächst aufwendig auf ein für die Nase passendes Format zugeschnitten werden müssen, wobei außerdem Gipspulver frei wird, dass den Operationsbereich verunreinigen kann. Weiterhin sind übliche Gipsbinden ausschließlich in weißer Farbe erhältlich, sodass das Aussehen der mit dem Gipsverband behandelten Person durch den auffallend weißen Gipsverband erheblich beeinträchtigt wird.

Als Alternative zu einem Gipsverband ist es bekannt, zur Ruhigstellung der Nase einen Verband aus einem thermoplastisch verformbaren Kunststoff zu verwenden. Ein solcher Verband, auch "Kunststoffgips" oder "Cast" genannt, hat den Vorteil, dass er bereits kurz nach dem Anlegen voll belastbar ist und eine höhere Bruchfestigkeit und Dauerbelastbarkeit als ein Gipsverband aufweist. Weiterhin sind Kunststoffverbände in vielen verschiedenen Farben erhältlich. Nachteilig ist jedoch, dass durch das schnelle Aushärten beim Anlegen des Verbands kaum Korrekturen seiner Form möglich sind. Durch eine nicht optimal angepasste Form kann es zu Druckstellen im Gesicht der behandelten Person kommen.

Die Druckschrift DE 201 05 609 U1 offenbart ein Colorierungssystem für Dentalgips. Um verschiedene Gipse voneinander zu unterscheiden, werden die Gipse mit unterschiedlichen Farben eingefärbt. Es werden keine Mittel beschrieben, die Vorbereitung eines Nasen-Gipsverbandes zu vereinfachen oder seine ästhetische Wirkung zu verbessern.

Die Druckschriften DE 836 550 C und DE 836 551 C offenbaren jeweils ein Verfahren zur Herstellung von Gipsbinden. Der Gips wird mit einem Farbstoff versehen, wobei die Farbe den Härtegrad der Gipsbinde anzeigt. Verschiedene Farbwerte zeigen verschiedene Härtegrade an. Es werden keine Mittel beschrieben, die Vorbereitung eines Nasen-Gipsverbandes zu vereinfachen oder seine ästhetische Wirkung zu verbessern. Die Druckschrift US2015073930 befasst sich mit der Einfärbung von Klebeverbänden, so dass die Farbe des Verbandes mit dem Hautton übereinstimmt.

### Technische Aufgabe

Die Aufgabe der Erfindung ist es, ein einfach durchzuführendes, zeiteffizientes und kostengünstiges Verfahren zur Vorbereitung von Gipsbinden für einen Nasen-Gipsverband zu schaffen, dass die ästhetische Wirkung des Gipsverbandes verbessert. Außerdem ist es die Aufgabe der Erfindung, ein kostengünstiges System zur Durchführung des Verfahrens zu schaffen.

### Technische Lösung

Der Gegenstand der vorliegenden Erfindung stellt ein Verfahren gemäß Anspruch 1 bereit, das die technische Aufgabe löst. Ebenso wird die Aufgabe durch ein System gemäß Anspruch 9 gelöst. Vorteilhafte Ausgestaltungen ergeben sich aus den abhängigen Ansprüchen.

### Beschreibung der Ausführungsarten

Die Erfindung betrifft ein Verfahren zur Vorbereitung von Gipsbinden für einen Gipsverband aus einer Mehrzahl von Gipsbinden-Lagen für eine menschliche Nase. Die Mehrzahl von Gipsbinden-Lagen ist vorzugsweise so gewählt, dass der Gipsverband eine ausreichende mechanische Stabilität zur Ruhigstellung der Nase erhält. Der Gipsverband kann beispielsweise 2 bis 10, insbesondere 4 bis 8, bevorzugt 6, Gipsbinden-Lagen umfassen.

Das Verfahren umfasst nur die Vorbereitung der Gipsbinden, nicht jedoch ein Aufbringen der Gipsbinden auf die Nase oder ein Modellieren des Gipsverbandes aus den Gipsbinden auf der Nase.

Im Sinne der Erfindung werden Binden zur Herstellung eines Verbands, die das Mineral Gips als Bindemittel enthalten, als Gipsbinden bezeichnet. Mit Kunstharz als Bindemittel versehene Kunststofffasern zur Herstellung von Kunststoffverbänden (sog. "Kunststoffgipse" oder "Casts") sind keine Gipsbinden im Sinne der Erfindung.

Das Verfahren umfasst ein Bereitstellen zumindest eines flächigen Gipsbinden-Grobzuschnitts und einer Einweichflüssigkeit, beispielsweise Wasser, insbesondere Leitungswasser, für den Gipsbinden-Grobzuschnitt, wobei der zumindest eine Gipsbinden-Grobzuschnitt die Mehrzahl von Gipsbinden-Lagen umfasst und ein an die Physiologie einer typischen Nase vorangepasstes Grob-Format aufweist.

Dadurch, dass der Gipsbinden-Grobzuschnitt ein vorangepasstes Grob-Format aufweist, kann er schneller und einfacher auf das für die Nase passende Format zugeschnitten werden oder ein Zuschneiden entfällt sogar ganz.

Dadurch, dass der Gipsbinden-Grobzuschnitt die Mehrzahl von Gipsbinden-Lagen umfasst, kann er besonders schnell und einfach weiterverarbeitet werden. Insbesondere ein Zuschneiden und/oder Einweichen der Gipsbinden-Lagen und/oder ein Aufbringen der Gipsbinden-Lagen auf die Nase kann jeweils für alle Gipsbinden-Lagen gemeinsam und somit besonders schnell und einfach erfolgen.

Das Verfahren umfasst ein Ermitteln einer Hautfarbe der Nase, umfassend einen Hautfarbton und eine Hautfarbsättigung.

Das Verfahren umfasst ein Auswählen zumindest eines Farbstoffs zum Färben des Gipsbinden-Grobzuschnitts in dem Hautfarbton. Bei dem zumindest einen Farbstoff kann es sich um einen einzigen Farbstoff oder um eine Mischung aus mehreren Farbstoffen handeln. Der zumindest eine Farbstoff umfasst vorzugsweise in Kosmetikfarbstoff und/oder einen Lebensmittelfarbstoff, um eine Hautverträglichkeit des gefärbten Gipsbinden-Grobzuschnitts sicherzustellen.

Das Verfahren umfasst ein Bestimmen einer zum Färben des Gipsbinden-Grobzuschnitts mit der Hautfarbsättigung notwendigen Farbstoffkonzentration des zumindest einen Farbstoffs in einem zum Durchtränken des Gipsbinden-Grobzuschnitts ausreichenden Durchtränkungsvolumen der Einweichflüssigkeit.

Das Verfahren umfasst ein Dosieren eines Flüssigkeitsvolumens der Einweichflüssigkeit und/oder einer Farbstoffmenge des zumindest einen Farbstoffs mit einer Dosiervorrichtung zur Erzeugung der Farbstoffkonzentration des zumindest einen Farbstoffs in dem Flüssigkeitsvolumen, wobei das Flüssigkeitsvolumen zumindest so groß ist wie das Durchtränkungsvolumen.

Das Flüssigkeitsvolumen und/oder die Farbstoffmenge werden also so dosiert, dass die gewünschte Farbstoffkonzentration erzielt wird, und ein ausreichend großes Flüssigkeitsvolumen zum Durchtränken des Gipsbinden-Grobzuschnitts zur Verfügung steht.

Durchtränken der Mehrzahl von Gipsbinden-Lagen des Gipsbinden-Grobzuschnitts in dem Durchtränkungsvolumen der Einweichflüssigkeit mit der Farbstoffkonzentration des zumindest einen Farbstoffs.

Der Gipsbinden-Grobzuschnitt ist vorzugsweise so durchlässig für die Einweichflüssigkeit gestaltet, dass ein vollständiges Durchtränken in einer für Gipsbinden üblichen Einweichzeit, beispielsweise in 1 s bis 30 s, insbesondere in 2 s bis 15 s, bevorzugt in 5 s bis 10 s, möglich ist.

Durch das Durchtränken kann der zumindest eine Farbstoff in alle Gipsbinden-Lagen eindringen, sodass ein vollständig in der Hautfarbe durchgefärbter Gipsbinden-Grobzuschnitt entsteht. Mit diesem Gipsbinden-Grobzuschnitt kann dann ein in der Hautfarbe durchgefärbter Gipsverband auf die Nase modelliert werden. Durch die Färbung in der Hautfarbe ist der Gipsverband ästhetisch weniger störend als ein üblicher Gipsverband. Dadurch, dass der Gipsbinden-Grobzuschnitt durchgefärbt ist, wird sichergestellt, dass durch ein Modellieren des Gipsverbandes und/oder Abrieb einer Oberfläche des Gipsverbandes keine ungleichmäßige Färbung entsteht.

Das Ermitteln der Hautfarbe umfasst vorzugsweise ein Auswählen einer der Hautfarbe entsprechenden Referenzfarbe einer Farbskala. Das Auswählen des zumindest einen Farbstoffs und/oder das Bestimmen der Farbstoffkonzentration umfasst bevorzugt ein Auswählen zumindest eines der Referenzfarbe zugeordneten Farbstoffs und/oder einer der Referenzfarbe zugeordneten Farbstoffkonzentration.

Mit Hilfe der Farbskala kann die Hautfarbe besonders einfach und objektiv ermittelt werden. Die Zuordnung der Referenzfarbe zu dem zumindest einen Farbstoff und/oder der Farbstoffkonzentration kann beispielsweise in einer physischen oder elektronischen Tabelle oder einer Datenbank hinterlegt sein, aus der ein Nutzer des Verfahrens die Zuordnung ablesen oder abrufen kann.

Die Farbskala und eine physische Tabelle mit den Zuordnungen können beispielsweise auf einem gemeinsamen Träger, insbesondere einem Blatt Papier, einem Stück Karton, einer Schablone oder einem Aufkleber, angebracht sein, damit die einzelnen Auswahlschritte möglichst schnell und zuverlässig ausgeführt werden können.

Die Dosiervorrichtung umfasst vorzugsweise ein Einstellmittel zur Einstellung einer von der Dosiervorrichtung abgegebenen Farbstoffmenge des zumindest einen Farbstoffs. Die Dosierungsvorrichtung kann beispielsweise ein Desinfektionsmittelspender mit einstellbarer Abgabemenge ausgestaltet sein.

Das Dosieren umfasst vorzugsweise ein Dosieren der Farbstoffmenge des zumindest einen Farbstoffs mit der Dosiervorrichtung zur Einstellung der Farbstoffkonzentration des zumindest einen Farbstoffs in dem Flüssigkeitsvolumen. Vorzugsweise ist die Dosiervorrichtung zur Anzeige der sich ergebenden Farbstoffkonzentration ausgelegt, um Fehlbedienungen zu vermeiden. Das Einstellmittel kann beispielsweise eine Anzahl von Markierungen aufweisen, die jeweils einer Farbstoffkonzentration zugeordnet sind, wobei die Zuordnung insbesondere in einer Tabelle oder Datenbank zur Zuordnung von Referenzfarben zu Farbstoffkonzentrationen hinterlegt sein kann.

Insbesondere kann das Flüssigkeitsvolumen dem Durchtränkungsvolumen entsprechen, sodass möglichst wenig Einweichflüssigkeit und Farbstoff verbraucht werden.

Die dosierte Farbstoffmenge wird vorzugsweise dem Flüssigkeitsvolumen der Einweichflüssigkeit, beispielsweise in einer Schale zum Einweichen des Gipsbinden-Grobzuschnitts, zugegeben und insbesondere damit vermischt.

Die Dosiervorrichtung umfasst vorzugsweise ein Anzeigemittel zur Anzeige eines Flüssigkeitsvolumens der Einweichflüssigkeit. Die Dosiervorrichtung kann beispielsweise einen Behälter zur Aufnahme der Einweichflüssigkeit mit einer Skala zur Anzeige des in dem Behälter enthaltenen Flüssigkeitsvolumens, insbesondere einen Messbecher oder eine Messschale zum Einweichen des Gipsbinden-Grobzuschnitts, umfassen.

Vorzugsweise ist die Skala zur Anzeige der sich aus dem Flüssigkeitsvolumen und der Farbstoffmenge ergebenden Farbstoffkonzentration ausgelegt, um Fehlbedienungen zu vermeiden. Die Skala kann beispielsweise eine Anzahl von Markierungen aufweisen, die jeweils einer Farbstoffkonzentration zugeordnet sind, wobei die Zuordnung insbesondere in einer Tabelle oder Datenbank zur Zuordnung von Referenzfarben zu Farbstoffkonzentrationen hinterlegt sein kann.

Das Dosieren umfasst vorzugsweise ein Dosieren des Flüssigkeitsvolumens der Einweichflüssigkeit mit der Dosiervorrichtung zur Einstellung der Farbstoffkonzentration der Farbstoffmenge des zumindest einen Farbstoffs in dem Flüssigkeitsvolumen.

Vorzugsweise wird die Farbstoffmenge des zumindest einen Farbstoffs in dem Verfahren in die Dosiervorrichtung gegeben oder ist darin vorgelegt. Dadurch kann die Einweichflüssigkeit in der Dosiervorrichtung mit dem Farbstoff vermischt werden, ohne dass ein zusätzlicher Schritt notwendig wäre, um die Einweichflüssigkeit und den Farbstoff zu vermischen.

Das Verfahren umfasst vorzugsweise ein Ausmessen eines für den Gipsverband an der Nase notwendigen Zuschnitt-Formats des Gipsbinden-Grobzuschnitts und ein gemeinsames Zuschneiden der Mehrzahl von Gipsbinden-Lagen des Gipsbinden-Grobzuschnitts zu einem Gipsbinden-Zuschnitt in dem Zuschnitt-Format. So werden die Gipsbinden-Lagen besonders schnell auf das Zuschnitt-Format gebracht.

Das Zuschneiden erfolgt vorzugsweise vor dem Durchtränken, da die Gipsbinden im trockenen Zustand einfacher zu schneiden sind, und nach dem Durchtränken nur wenig Zeit bis zum Aushärten des Gipses zur Verfügung steht.

Das Bereitstellen der Mehrzahl von Gipsbinden-Lagen des Gipsbinden-Grobzuschnitts erfolgt vorzugsweise in einer gemeinsamen Folienverpackung, beispielsweise aus Papier und/oder einer Kunststofffolie. Die Folienverpackung ist vorzugsweise so gestaltet, dass sie zusammen mit dem darin enthaltenen Gipsbinden-Grobzuschnitt mit einer üblichen Verbandschere durchgeschnitten werden kann.

Das Zuschneiden der Mehrzahl von Gipsbinden-Lagen des Gipsbinden-Grobzuschnitts erfolgt vorzugsweise in der Folienverpackung. Dadurch wird bei dem Zuschneiden weniger Gips freigesetzt, der den Operationsbereich verunreinigen könnte.

Das Verfahren umfasst vorzugsweise ein Aufbringen, bevorzugt ein Aufkleben, einer Schablone auf die Nase. Das Ausmessen umfasst vorzugsweise ein Markieren einer Kontur der Nase auf der auf die Nase aufgebrachten Schablone. Das Verfahren umfasst vorzugsweise ein Aufbringen, bevorzugt ein Aufkleben, der Schablone mit der markierten Kontur auf den Gipsbinden-Grobzuschnitt, bevorzugt auf eine Folienverpackung des Gipsbinden-Grobzuschnitts. Das Zuschneiden der Mehrzahl von Gipsbinden-Lagen des Gipsbinden-Grobzuschnitts erfolgt vorzugsweise entlang der Kontur.

Mit den vorgenannten Schritten ist es besonders schnell, einfach und zuverlässig möglich, die Gipsbinden-Lagen im für die Nase passenden Zuschnitt-Format zuzuschneiden. Insbesondere ist es möglich, dass eine erste Person, beispielsweise ein Arzt, nur das zur Festlegung des Zuschnitt-Formats kritische Markieren durchführt, und eine zweite Person, beispielsweise ein Arzthelfer, das Aufbringen und das Zuschneiden durchführt, sodass sich die erste Person währenddessen um andere Aufgaben kümmern kann.

Das Verfahren umfasst vorzugsweise ein Vor-Färben der Gipsbinden in einer universellen Haut-Grundfarbe vor den übrigen Schritten des Verfahrens, wobei die Haut-Grundfarbe für einen bestimmten Hautfarbton oder für mehrere Hautfarbtöne universell sein kann. Durch das Vor-Färben kann das Färben während des Durchtränkens der Gipsbinden schneller und/oder mit weniger Farbstoff erfolgen.

Die Erfindung betrifft ein System zur Durchführung eines erfindungsgemäßen Verfahrens zur Vorbereitung einer Modellierung eines Gipsverbandes aus einer Mehrzahl von Gipsbinden-Lagen für eine menschliche Nase.

Das System umfasst zumindest einen flächigen Gipsbinden-Grobzuschnitt, bevorzugt in einer Folienverpackung, wobei der zumindest eine Gipsbinden-Grobzuschnitt die Mehrzahl von Gipsbinden-Lagen umfasst und ein an die Physiologie einer typischen Nase vorangepasstes Grob-Format aufweist.

Das System umfasst zumindest einen Farbstoff zum Färben des Gipsbinden-Grobzuschnitts in einem Hautfarbton der Nase.

Das System umfasst zumindest eine Dosiervorrichtung zum Dosieren eines Flüssigkeitsvolumens einer Einweichflüssigkeit für den Gipsbinden-Grobzuschnitt und/oder einer Farbstoffmenge des zumindest einen Farbstoffs.

Die Komponenten des Systems können insbesondere so ausgestaltet sein, wie zuvor im Zusammenhang mit dem Verfahren beschrieben, woraus sich die dort genannten Vorteile ergeben.

Das System umfasst vorzugsweise zumindest eine Farbskala mit einer Anzahl von Referenzfarben zum Ermitteln einer Hautfarbe der Nase, umfassend einen Hautfarbton und eine Hautfarbsättigung.

Die Dosiervorrichtung umfasst vorzugsweise ein Einstellmittel zur Einstellung einer von der Dosiervorrichtung abgegebenen Farbstoffmenge des zumindest einen Farbstoffs umfasst, und/oder ein Anzeigemittel zur Anzeige eines Flüssigkeitsvolumens der Einweichflüssigkeit.

Das System umfasst vorzugsweise eine Schablone zum Aufbringen, bevorzugt zum Aufkleben, auf die Nase und zum Markieren einer Kontur der Nase auf der auf die Nase aufgebrachten Schablone.

Die Schablone umfasst bevorzugt eine Farbskala mit einer Anzahl von Referenzfarben zum Ermitteln einer Hautfarbe der Nase, umfassend einen Hautfarbton und eine Hautfarbsättigung. Durch die Integration der Farbskala in die Schablone können das Markieren der Kontur und das Ermitteln der Hautfarbe besonders schnell hintereinander durchgeführt werden. Das System umfasst dadurch außerdem besonders wenige Einzelkomponenten, sodass es besonders kostengünstig herstellbar und einfach verwendbar ist.

Das System umfasst vorzugsweise eine Anzahl von Hilfs-Markierungen, bevorzugt Schnittmustern, Anlegemarken und/oder Symmetrielinien, auf dem Gipsbinden-Grobzuschnitt, einer Folienverpackung des Gipsbinden-Grobzuschnitts und/oder einer Schablone zum Aufbringen auf die Nase für ein Markieren einer Kontur der Nase auf dem Gipsbinden-Grobzuschnitt, der Folienverpackung und/oder der Schablone.

Die Schnittmuster können beispielsweise typische Nasenkonturen abhängig von einer Körpergröße, einem Alter und/oder einem Geschlecht eines Menschen darstellen.

Die Hilfs-Markierungen erlauben eine besonders einfache, schnelle und zuverlässige Markierung der Kontur der Nase.

Die Erfindung betrifft eine Verwendung eines erfindungsgemäßen Systems zur Durchführung eines erfindungsgemäßen Verfahrens.

### Kurze Beschreibung der Zeichnungen

Weitere Vorteile, Ziele und Eigenschaften der Erfindung werden anhand nachfolgender Beschreibung und anliegender Zeichnungen erläutert, in welchen beispielhaft erfindungsgemäße Gegenstände dargestellt sind. Merkmale, welche in den Figuren wenigstens im Wesentlichen hinsichtlich ihrer Funktion übereinstimmen, können hierbei mit gleichen Bezugszeichen gekennzeichnet sein, wobei diese Merkmale nicht in allen Figuren beziffert und erläutert sein müssen.

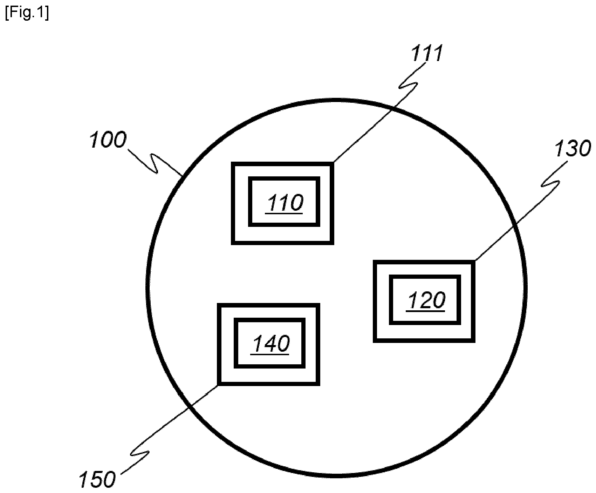

Figur 1 zeigt schematisch ein erfindungsgemäßes System.

### Fig.1

Figur 1 zeigt schematisch ein erfindungsgemäßes System 100.

Das gezeigte System 100 umfasst einen flächigen Gipsbinden-Grobzuschnitt 110, bevorzugt in einer Folienverpackung 111, wobei der zumindest eine Gipsbinden-Grobzuschnitt 110 eine Mehrzahl von, beispielsweise sechs, Gipsbinden-Lagen umfasst und ein an die Physiologie einer typischen Nase vorangepasstes Grob-Format aufweist.

Das gezeigte System 100 umfasst einen Farbstoff 120, beispielsweise einen Kosmetikfarbstoff, zum Färben des Gipsbinden-Grobzuschnitts 110 in einem Hautfarbton der Nase und eine Dosiervorrichtung 130 zum Dosieren einer Farbstoffmenge des Farbstoffs 120.

Die Dosiervorrichtung 130 kann beispielsweise den Farbstoff 120 enthalten und insbesondere ein Einstellmittel zur Einstellung einer von der Dosiervorrichtung 130 abgegebenen Farbstoffmenge des Farbstoffs 120 umfassen. Die Dosiervorrichtung 130 kann beispielsweise wie ein einstellbarer Desinfektionsmittelspender aufgebaut sein.

Das gezeigte System 100 umfasst eine Farbskala 140 mit einer Anzahl von Referenzfarben zum Ermitteln einer Hautfarbe der Nase, umfassend einen Hautfarbton und eine Hautfarbsättigung.

Das gezeigte System 100 umfasst eine Schablone 150 zum Aufbringen, bevorzugt zum Aufkleben, auf die Nase und zum Markieren einer Kontur der Nase auf der auf die Nase aufgebrachten Schablone 150, wobei die Schablone 150 vorzugsweise die Farbskala 140 umfasst.

**Liste der Bezugszeichen**

| | | | |
|---|---|---|---|
| 100 | System | 130 | Dosiervorrichtung |
| 110 | Gipsbinden-Grobzuschnitt | 140 | Farbskala |
| 111 | Folienverpackung | 150 | Schablone |
| 120 | Farbstoff | | |

## Patentansprüche

1. Verfahren zur Vorbereitung von Gipsbinden für einen Gipsverband aus einer Mehrzahl von Gipsbinden-Lagen für eine menschliche Nase, **gekennzeichnet durch folgende Schritte:**
a. Bereitstellen zumindest eines flächigen Gipsbinden-Grobzuschnitts (110) und einer Einweichflüssigkeit für den Gipsbinden-Grobzuschnitt (110),
i. wobei der zumindest eine Gipsbinden-Grobzuschnitt (110) die Mehrzahl von Gipsbinden-Lagen umfasst und
ii. ein an die Physiologie einer typischen Nase vorangepasstes Grob-Format aufweist;
b. Ermitteln einer Hautfarbe der Nase, umfassend einen Hautfarbton und eine Hautfarbsättigung;
c. Auswählen zumindest eines Farbstoffs (120) zum Färben des Gipsbinden-Grobzuschnitts (110) in dem Hautfarbton;
d. Bestimmen einer zum Färben des Gipsbinden-Grobzuschnitts (110) mit der Hautfarbsättigung notwendigen Farbstoffkonzentration des zumindest einen Farbstoffs (120) in einem zum Durchtränken des Gipsbinden-Grobzuschnitts (110) ausreichenden Durchtränkungsvolumen der Einweichflüssigkeit;
e. Dosieren eines Flüssigkeitsvolumens der Einweichflüssigkeit und/oder einer Farbstoffmenge des zumindest einen Farbstoffs (120) mit einer Dosiervorrichtung (130) zur Erzeugung der Farbstoffkonzentration des zumindest einen Farbstoffs (120) in dem Flüssigkeitsvolumen,
i. wobei das Flüssigkeitsvolumen zumindest so groß ist wie das Durchtränkungsvolumen, und
f. Durchtränken der Mehrzahl von Gipsbinden-Lagen des Gipsbinden-Grobzuschnitts (110) in dem Durchtränkungsvolumen der Einweichflüssigkeit mit der Farbstoffkonzentration des zumindest einen Farbstoffs (120).

2. Verfahren nach Anspruch 1,
**dadurch gekennzeichnet, dass**
a. das Ermitteln der Hautfarbe ein Auswählen einer der Hautfarbe entsprechenden Referenzfarbe einer Farbskala (140) umfasst; und
b. das Auswählen des zumindest einen Farbstoffs (120) und/oder das Bestimmen der Farbstoffkonzentration ein Auswählen zumindest eines der Referenzfarbe zugeordneten Farbstoffs (120) und/oder einer der Referenzfarbe zugeordneten Farbstoffkonzentration umfasst.

3. Verfahren nach Anspruch 1 oder 2,
**dadurch gekennzeichnet, dass**
a. die Dosiervorrichtung (130) ein Einstellmittel zur Einstellung einer von der Dosiervorrichtung (130) abgegebenen Farbstoffmenge des zumindest einen Farbstoffs (120) umfasst,
b. wobei das Dosieren ein Dosieren der Farbstoffmenge des zumindest einen Farbstoffs (120) mit der Dosiervorrichtung (130) zur Einstellung der Farbstoffkonzentration des zumindest einen Farbstoffs (120) in dem Flüssigkeitsvolumen umfasst.

4. Verfahren nach einem der Ansprüche 1 oder 2,
**dadurch gekennzeichnet, dass**
a. die Dosiervorrichtung (130) ein Anzeigemittel zur Anzeige eines Flüssigkeitsvolumens der Einweichflüssigkeit umfasst,
b. wobei das Dosieren ein Dosieren des Flüssigkeitsvolumens der Einweichflüssigkeit mit der Dosiervorrichtung (130) zur Einstellung der Farbstoffkonzentration der Farbstoffmenge des zumindest einen Farbstoffs (120) in dem Flüssigkeitsvolumen umfasst.

5. Verfahren nach einem der Ansprüche 1 bis 4,
**gekennzeichnet durch folgende Schritte:**
a. Ausmessen eines für den Gipsverband an der Nase notwendigen Zuschnitt-Formats des Gipsbinden-Grobzuschnitts (110) und
b. gemeinsames Zuschneiden der Mehrzahl von Gipsbinden-Lagen des Gipsbinden-Grobzuschnitts (110) zu einem Gipsbinden-Zuschnitt in dem Zuschnitt-Format,
c. wobei das Zuschneiden vor dem Durchtränken erfolgt.

6. Verfahren nach Anspruch 5,
**dadurch gekennzeichnet, dass**
a. das Bereitstellen der Mehrzahl von Gipsbinden-Lagen des Gipsbinden-Grobzuschnitts (110) in einer gemeinsamen Folienverpackung (111) erfolgt, und
b. das Zuschneiden der Mehrzahl von Gipsbinden-Lagen des Gipsbinden-Grobzuschnitts (110) in der Folienverpackung (111) erfolgt.

7. Verfahren nach Anspruch 5 oder 6,
**dadurch gekennzeichnet, dass**
a. das Verfahren ein Aufbringen, bevorzugt ein Aufkleben, einer Schablone (150) auf die Nase umfasst,
b. das Ausmessen ein Markieren einer Kontur der Nase auf der auf die Nase aufgebrachten Schablone (150) umfasst,
c. das Verfahren ein Aufbringen, bevorzugt ein Aufkleben, der Schablone (150) mit der markierten Kontur auf den Gipsbinden-Grobzuschnitt (110), bevorzugt auf eine Folienverpackung (111) des Gipsbinden-Grobzuschnitts (110), umfasst, und
d. das Zuschneiden der Mehrzahl von Gipsbinden-Lagen des Gipsbinden-Grobzuschnitts (110) entlang der Kontur erfolgt.

8. Verfahren nach einem der Ansprüche 1 bis 7,
**gekennzeichnet durch den Schritt:**
Vor-Färben der Gipsbinden in einer universellen Haut-Grundfarbe vor den übrigen Schritten des Verfahrens.

9. System (100) zur Durchführung eines Verfahrens nach einem der Ansprüche 1 bis 8 zur Vorbereitung einer Modellierung eines Gipsverbandes aus einer Mehrzahl von Gipsbinden-Lagen für eine menschliche Nase, **gekennzeichnet durch**
a. zumindest einen flächigen Gipsbinden-Grobzuschnitt (110), bevorzugt in einer Folienverpackung (111),
i. wobei der zumindest eine Gipsbinden-Grobzuschnitt (110) die Mehrzahl von Gipsbinden-Lagen umfasst und
ii. ein an die Physiologie einer typischen Nase vorangepasstes Grob-Format aufweist;
b. zumindest einen Farbstoff (120) zum Färben des Gipsbinden-Grobzuschnitts (110) in einem Hautfarbton der Nase und
c. zumindest eine Dosiervorrichtung (130) zum Dosieren eines Flüssigkeitsvolumens einer Einweichflüssigkeit für den Gipsbinden-Grobzuschnitt (110) und/oder einer Farbstoffmenge des zumindest einen Farbstoffs (120).

10. System (100) nach Anspruch 9,
**gekennzeichnet durch**
a. zumindest eine Farbskala (140) mit einer Anzahl von Referenzfarben zum Ermitteln einer Hautfarbe der Nase, umfassend einen Hautfarbton und eine Hautfarbsättigung.

11. System (100) nach Anspruch 9 oder 10,
**dadurch gekennzeichnet, dass** die Dosiervorrichtung (130)
a. ein Einstellmittel zur Einstellung einer von der Dosiervorrichtung (130) abgegebenen Farbstoffmenge des zumindest einen Farbstoffs (120) umfasst, und/oder
b. ein Anzeigemittel zur Anzeige eines Flüssigkeitsvolumens der Einweichflüssigkeit umfasst.

12. System (100) nach einem der Ansprüche 9 bis 11,
**gekennzeichnet durch**
eine Schablone (150) zum Aufbringen, bevorzugt zum Aufkleben, auf die Nase und zum Markieren einer Kontur der Nase auf der auf die Nase aufgebrachten Schablone (150).

13. System (100) nach Anspruch 12,
**dadurch gekennzeichnet, dass**
die Schablone (150) eine Farbskala (140) mit einer Anzahl von Referenzfarben zum Ermitteln einer Hautfarbe der Nase, umfassend einen Hautfarbton und eine Hautfarbsättigung, umfasst.

14. System (100) nach einem der Ansprüche 9 bis 13,
**gekennzeichnet durch**
eine Anzahl von Hilfs-Markierungen, bevorzugt Schnittmustern, Anlegemarken und/oder Symmetrielinien, auf dem Gipsbinden-Grobzuschnitt (110), einer Folienverpackung (111) des Gipsbinden-Grobzuschnitts (110) und/oder einer Schablone (150) zum Aufbringen auf die Nase für ein Markieren einer Kontur der Nase auf dem Gipsbinden-Grobzuschnitt (110), der Folienverpackung (111) und/oder der Schablone (150).

15. Verwendung eines Systems (100) nach einem der Ansprüche 9 bis 14 zur
Durchführung eines Verfahrens nach einem der Ansprüche 1 bis 8.

## Claims

1. A method of preparing plaster bandages for a plaster cast from a plurality of plaster bandage layers for a human nose,
**characterized by** the following steps:
a. providing at least one flat plaster bandage coarse blank (110) and a soaking liquid for the plaster bandage coarse blank (110),
i. wherein the at least one plaster bandage coarse blank (110) comprises the plurality of plaster bandage layers, and
ii. has a coarse format preadapted to the physiology of a typical nose,
b. determining a skin colour of the nose comprising a skin colour tone and a skin colour saturation,
c. selecting at least one dye (120) to dye plaster bandage coarse blank (110) in the skin colour tone,
d. determining a dye concentration of the at least one dye (120) necessary for dyeing the plaster bandage coarse blank (110) with the skin colour saturation in an impregnation volume of the soaking liquid sufficient to impregnate the plaster bandage coarse blank (110),
e. metering a liquid volume of the soaking liquid and/or a dye quantity of the at least one dye (120) with a metering device (130) for generating the dye concentration of the at least one dye (120) in the liquid volume,
i. wherein the liquid volume is at least as large as the impregnation volume, and
f. impregnating the plurality of plaster bandage layers of the plaster bandage coarse blank (110) in the impregnation volume of the soaking liquid with the dye concentration of the at least one dye (120).

2. The method according to claim 1,
**characterized in that**
a. the step of determining the skin colour comprises selecting a reference colour of a colour scale (140) corresponding to the skin colour; and
b. the step of selecting the at least one dye (120) and/or determining the dye concentration comprises selecting at least one dye (120) assigned to the reference colour and/or selecting a dye concentration assigned to the reference colour.

3. The method according to claim 1 or 2,
**characterized in that**
a. the metering device (130) comprises an adjusting means for adjusting an amount of dye of the at least one dye (120) dispensed by the metering device (130),
b. wherein the step of metering comprises metering the amount of dye of the at least one dye (120) with the metering device (130) to adjust the dye concentration of the at least one dye (120) in the liquid volume.

4. The method according to one of claims 1 or 2,
**characterized in that**
a. the metering device (130) comprises an indicator means for indicating a liquid volume of the soaking liquid,
b. wherein the step of metering comprises metering the liquid volume of the soaking liquid with the metering device (130) to adjust the dye concentration of the dye amount of the at least one dye (120) in the liquid volume.

5. The method according to one of claims 1 to 4,
**characterized by** the following steps:
a. sizing of a blank format of the plaster bandage coarse blank (110) necessary for the plaster cast on the nose; and
b. jointly cutting the plurality of plaster bandage layers of the plaster bandage coarse blank (110) into a plaster bandage blank in the blank format,
c. wherein the step of cutting takes place before the step of impregnating.

6. The method according to claim 5,
**characterized in that**
a. the step of providing the plurality of plaster bandage layers of the plaster bandage coarse blank (110) takes place in a common film packaging (111), and
b. the step of cutting the plurality of plaster bandage layers of the plaster bandage coarse blank (110) takes place in the film packaging (111).

7. The method according to claim 5 or 6,
**characterized in that**
a. the method comprises applying, preferably gluing, a template (150) to the nose,
b. the step of measuring comprises marking a contour of the nose on the template (150) applied to the nose,
c. the method comprises applying, preferably gluing, the template (150) with the marked contour to the plaster bandage coarse blank (110), preferably to a film packaging (111) of the plaster bandage coarse blank (110), and
d. the step of cutting the plurality of plaster bandage layers of the plaster bandage coarse blank (110) takes place along the contour.

8. The method according to any one of claims 1 to 7,
**characterized by** the step of:
pre-colouring the plaster bandages in a universal basic skin colour before the remaining steps of the method.

9. A system (100) for carrying out a method according to one of claims 1 to 8 for preparing a modelling of a plaster cast from a plurality of plaster bandage layers for a human nose,
**characterized by**
a. at least one flat plaster bandage coarse blank (110), preferably in a film packaging (111), wherein the at least one plaster bandage coarse blank (110)
i. comprises the plurality of plaster bandage layers, and
ii. has a coarse format preadapted to the physiology of a typical nose,
b. at least one dye (120) for dyeing the plaster bandage coarse blank (110) in a skin colour tone of the nose and
c. at least one metering device (130) for metering a liquid volume of a soaking liquid for the plaster bandage coarse blank (110) and/or a quantity of dye of the at least one dye (120).

10. The system (100) of claim 9,
**characterized by**
at least one colour scale (140) having a number of reference colours for determining a skin colour of the nose, the skin colour comprising a skin colour tone and a skin colour saturation.

11. The system (100) according to claim 9 or 10,
**characterized in that** the metering device (130)
a. comprises an adjusting means for adjusting an amount of dye of the at least one dye (120) dispensed by the metering device (130), and/or
b. an indicating means for indicating a liquid volume of the soaking liquid.

12. The system (100) according to one of claims 9 to 11,
**characterized by**
a template (150) for applying, preferably for gluing, to the nose and for marking a contour of the nose on the template (150) applied to the nose.

13. The system (100) of claim 12,
**characterized in that**
the template (150) comprises a colour scale (140) with a number of reference colours for determining a skin colour of the nose, the skin colour comprising a skin colour tone and a skin colour saturation.

14. The system (100) according to any of claims 9 to 13,
**characterized by**
a number of auxiliary markings, preferably cutting patterns, contact marks and/or symmetry lines, on the plaster bandage coarse blank (110), on a film packaging (111) of the plaster bandage coarse blank (110) and/or on a template (150) for application to the nose for marking a contour of the nose on the plaster bandage coarse blank (110), on the film package (111) and/or on the template (150).

15. Use of a system (100) according to one of claims 9 to 14 for carrying out a method according to one of claims 1 to 8.

## Revendications

1. Procédé de préparation de bandes de plâtre pour un plâtre à partir d'une pluralité de couches de bande de plâtre pour un nez humain,
**caractérisé par** les étapes suivantes :
a. fournir au moins une coupe grossière de bande de plâtre (110) plate et un liquide de trempage pour la coupe grossière de bande de plâtre (110),
i. l'au moins une coupe grossière de bandage en plâtre (110) comprenant la pluralité de couches de bande de plâtre et
ii. ayant un format grossier pré-adapté à la physiologie d'un nez typique ;
b. déterminer une couleur de peau du nez comprenant un teint de peau et une saturation de couleur de peau ;
c. sélectionner au moins un colorant (120) pour colorer la coupe grossière de bande de plâtre (110) dans le teint de la peau ;
d. déterminer une concentration de colorant du au moins un colorant (120) nécessaire pour colorer la coupe grossière de bande de plâtre (110) avec la saturation de couleur de peau dans un volume de trempage du liquide de trempage suffisant pour tremper la coupe grossière de bande de plâtre (110) ;
e. doser un volume de liquide du liquide de trempage et/ou une quantité de colorant du au moins un colorant (120) avec un dispositif de dosage (130) pour générer la concentration de colorant du au moins un colorant (120) dans le volume de liquide,
i. le volume de liquide étant au moins aussi grand que le volume de trempage, et
f. tremper la pluralité de couches de bande de plâtre de la coupe grossière de bande de plâtre (110) dans le volume de trempage du liquide de trempage avec la concentration de colorant du au moins un colorant (120).

2. Procédé selon la revendication 1,
**caractérisé en ce que**
a. l'étape de déterminer la couleur de peau comprend la sélection d'une couleur de référence correspondant à la couleur de peau à partir d'une échelle de couleurs (140) ; et
b. l'étape de sélectionner l'au moins un colorant (120) et/ou l'étape de déterminer la concentration de colorant comprend une sélection d'au moins un colorant (120) affecté à la couleur de référence et/ou une concentration de colorant affectée à la couleur de référence.

3. Procédé selon la revendication 1 ou 2,
**caractérisé en ce que**
a. le dispositif de dosage (130) comprend un moyen de réglage pour régler une quantité de colorant du au moins un colorant (120) libéré par le dispositif de dosage (130),
b. l'étape de doser comprenant un dosage de la quantité de colorant du au moins un colorant (120) avec le dispositif de dosage (130) pour ajuster la concentration en colorant du au moins un colorant (120) dans le volume de liquide.

4. Procédé selon l'une des revendications 1 ou 2,
**caractérisé en ce que**
a. le dispositif de dosage (130) comprend un moyen d'affichage pour afficher un volume liquide du liquide de trempage,
b. l'étape de doser comprenant un dosage du volume de liquide du liquide de trempage avec le dispositif de dosage (130) pour ajuster la concentration en colorant de la quantité de colorant du au moins un colorant (120) dans le volume de liquide.

5. Procédé selon l'une des revendications 1 à 4,
**caractérisé par** les étapes suivantes :
a. mesurer un format de coupe de la coupe grossière de bande de plâtre (110) nécessaire pour le plâtre sur le nez et
b. découper ensemble la pluralité de couches de bande de plâtre de la coupe grossière de bande de plâtre (110) pour former une coupe de bande de plâtre dans le format de coupe,
c. l'étape de découper se faisant avant l'étape de tremper.

6. Procédé selon la revendication 5,
**caractérisé en ce que**
a. l'étape de fournir la pluralité de couches de bande de plâtre de la coupe grossière de bande de plâtre (110) s'effectue dans un l'emballage en film (111) commun, et
b. l'étape de découper la pluralité de couches de bande de plâtre de la coupe grossière de bande de plâtre (110) a lieu dans l'emballage en film (111).

7. Procédé selon la revendication 5 ou 6,
**caractérisé en ce que**
a. le procédé comprend une application, de préférence un collage, d'un modèle (150) sur le nez,
b. l'étape de mesurer comprenant un marquage d'un contour du nez sur le modèle (150) appliqué sur le nez,
c. le procédé comprenant une application, de préférence un collage, du modèle (150) avec le contour marqué sur la coupe grossière de bande de plâtre (110), de préférence sur un emballage en film (111) de la coupe grossière de bande de plâtre (110), et
d. l'étape de découper la pluralité de couches de bande de plâtre de la coupe grossière de bande de plâtre (110) s'effectuant le long du contour.

8. Procédé selon l'une des revendications 1 à 7,
**caractérisé par** l'étape suivante :
pré-teindre les bandes de plâtre dans une couleur de base universelle de peau avant les étapes restantes du procédé.

9. Système (100) pour la mise en oeuvre d'un procédé selon l'une des revendications 1 à 8 pour la préparation d'un modelage d'un plâtre à partir d'une pluralité de couches de bande de plâtre pour un nez humain,
**caractérisé par**
a. au moins une coupe grossière de bande de plâtre (110), de préférence dans un emballage en film (111), l'au moins une coupe grossière de bandage de plâtre (110)
i. comprenant la pluralité de couches de bande de plâtre et
ii. ayant un format grossier pré-adapté à la physiologie d'un nez typique ;
b. au moins un colorant (120) pour colorer la coupe grossière de bande de plâtre (110) dans un teint de couleur de peau du nez et
c. au moins un dispositif de dosage (130) pour doser un volume de liquide d'un liquide de trempage pour la coupe grossière de bande de plâtre (110) et/ou pour doser une quantité de colorant du au moins un colorant (120).

10. Système (100) selon la revendication 9,
**caractérisé par**
au moins une échelle de couleurs (140) avec un certain nombre de couleurs de référence pour déterminer une couleur de peau du nez, la couleur de peau comprenant un teint de peau et une saturation de couleur de peau.

11. Système (100) selon la revendication 9 ou 10,
**caractérisé en ce que**
le dispositif de dosage (130) comprend
a. un moyen de réglage pour régler une quantité de colorant du au moins un colorant (120) libéré par le dispositif de dosage (130), et/ou
b. un moyen d'affichage pour afficher un volume liquide du liquide de trempage.

12. Système (100) selon l'une quelconque des revendications 9 à 11,
**caractérisé par**
un modèle (150) à appliquer, de préférence à coller, sur le nez et à marquer un contour du nez sur le modèle (150) appliqué sur le nez.

13. Système (100) selon la revendication 12,
**caractérisé en ce que**
le modèle (150) comprend une échelle de couleurs (140) avec un certain nombre de couleurs de référence pour déterminer une couleur de peau du nez, la couleur de peau comprenant un teint de peau et une saturation de couleur de peau.

14. Système (100) selon l'une quelconque des revendications 9 à 13,
**caractérisé par**
un certain nombre de marquages auxiliaires, de préférence des motifs de découpe, des repères de positionnement et/ou des lignes de symétrie, sur la coupe grossière de bande de plâtre (110), sur un emballage en film (111) de la coupe grossière de bande de plâtre (110) et/ou sur un modèle (150) à appliquer sur le nez pour marquer un contour du nez sur la coupe grossière de bande de plâtre (110), sur l'emballage en film (111) et/ou sur le modèle (150).

15. Utilisation d'un système (100) selon l'une des revendications 9 à 14 pour la mise en oeuvre d'un procédé selon l'une des revendications 1 à 8.
